# EUROPÄISCHE PATENTSCHRIFT

(11) **EP 0 917 079 B1**
(45) Veröffentlichungstag und Bekanntmachung des Hinweises auf die Patenterteilung: **27.05.2015**
(21) Anmeldenummer: 98120907.5
(22) Anmeldetag: 04.11.1998
(51) Int. Cl.: G06F 19/00

(54) **Datenverarbeitungseinheit und Verfahren zur Verarbeitung von Daten**
Data processing unit and method for data processing
Unité et procédé de traitement de données

(30) Priorität: 07.11.1997 DE 19749205
(43) Veröffentlichungstag der Anmeldung: 19.05.1999
(73) Patentinhaber: Morpho Cards GmbH, 24220 Flintbek (DE); Medtronic Inc., 6229 GW Maastricht (NL)
(72) Erfinder: Zöllner, Heinz, 33104 Paderborn (DE); Hinssen, Hans-Josef, 59494 Soest (DE)
(74) Vertreter: Richardt Patentanwälte PartG mbB

(56) Entgegenhaltungen:
- EP-A- 0 730 882
- WO-A-97/04712
- DE-A- 4 213 797

## Beschreibung

Die Erfindung betrifft ein Datengerät zur telemetrischen Einstellung eines Implantats und/oder zur telemetrischen Kommunikation zwischen dem Implantat und dem Datengerät.

Es ist bereits bekannt, die technischen Daten eines Implantats und die patientenabhängigen Daten über die Krankheitsgeschichte des Patienten auf einer Chipkarte abzuspeichern, so dass dem jeweiligen behandelnden Arzt bei jeder nach der Implantation erfolgenden Nachuntersuchung die aktuellen Daten zur Verfügung stehen. Die entsprechenden Daten werden in ein Datengerät manuell eingegeben und über ein mit dem Datengerät verbundenes Kartenlesegerät auf der Chipkarte gespeichert. Das Auslesen der Daten aus der Chipkarte erfolgt in umgekehrter Richtung.

Nachteilig an der bekannten Verarbeitung zur Datenübertragung ist, dass keine automatische Abspeicherung der Daten auf einem dem Patienten auszuhändigenden Datenträger erfolgt. Eine automatische Abrechnung des Implantats zwischen dem Klinikträger und dem Implantathersteller ist nicht vorgesehen ist.

Weiterhin ist es bekannt, Implantate wie beispielsweise Herzschrittmacher auf telemetrischem Wege einzustellen und die entsprechenden Zustandsdaten des Implantats herauszulesen.

In EP 0 730 882 wird ein System zur Stimulierung des Herzens eines Patienten beschrieben, das aus einem Implantat mit einem Mikroprozessor und einem Datengerät besteht. Das Implantat enthält einen Speicher, in dem im Wesentlichen alle Informationen gespeichert werden, die notwendig sind, das Implantat zu programmieren. Diese Informationen werden auf telemetrischem Wege zum Datengerät übertragen und dort angezeigt. Dort ggf. neu erstellte Programme können zum Implantat zurück übertragen werden. Auf diese Weise kann jedes mögliche Datengerät eingesetzt werden, ohne dass dem Datengerät von außen zusätzliche Informationen zur Verfügung gestellt werden müssen. In dem Datengerät sind keine personen- oder implantats-spezifischen Daten gespeichert. Dies hat zur Folge, dass der Patient nur bei dem Arzt behandelt werden kann, der das Datengerät zur Verfügung hat, auf dem die personen- oder implantats-spezifischen Daten gespeichert sind.

Aufgabe der Erfindung ist es daher, ein Datengerät zur telemetrischen Einstellung eines Implantats und/oder zur telemetrischen Kommunikation zwischen dem Implantat und dem Datengerät anzugeben, welche einerseits eine Speicherung, Verarbeitung und gegebenenfalls Modifikation von patientenbezogenen Daten (Zustandsdaten des Implantats) und andererseits eine Bereitstellung und nicht-flüchtige Speicherung von implantatsbezogenen Daten (Verwaltungsdaten des Implantats) gestattet, so dass die Daten auch nach Abschaltung des Datengerätes bzw. ohne Zwang der Nutzung eines bestimmten Datengerätes erhalten bleiben.

Zur Lösung dieser Aufgabe weist die Erfindung die Merkmale des Patentanspruchs 1 auf.

Das erfindungsgemässe Datengerätermöglicht auf einfache Weise die Bereitstellung von auf einem tragbaren Datenträger abgespeicherten Daten, wobei auf einem ersten Datenträger Zustandsdaten medizinischen bzw. technischen Inhalts abgespeichert sind zur Aushändigung an den Patienten und wobei auf einem zweiten Datenträger implantatsbezogene Verwaltungsdaten über das Implantat abgespeichert sind zum Verbleib im Datengerät bzw. zur Aushändigung an eine authorisierte Person. Vorzugsweise können die Verwaltungsdaten Abrechnungsinformationen enthalten, die regelmässig von einem Vertreter des Implantatsherstellers ausgelesen werden können. Dem behandelnden Arzt wird ein schneller Zugriff auf die aktuellen Einstellungsdaten des Implantats und gegebenenfalls zusätzlich auf die Daten des Patienten ermöglicht.

Vorteilhaft können auf dem ersten Datenträger alle patientenrelevanten Daten gespeichert werden, wobei zusätzlich Messdaten, die durch zyklische Messungen des Implantats selbst ermittelt worden sind, dann bei einer Nachuntersuchung des Patienten mit den bisherigen auf dem Datenträger gespeicherten Patientendaten, die ein vorheriges Zeitintervall repräsentieren, verglichen und ausgewertet werden können. Diese Auswertung erfolgt vorzugsweise in dem Datengerät.

Der zweite Datenträger sammelt die Verwaltungsdaten mehrerer Implantate und ermöglicht somit eine definierte und körperlich getrennte Aufstellung von Implantaten zur späteren ortsungebundenen Auswertung derselben. Dabei wird durch das in dem Datengerät implemetierte Steuerprogramm sichergestellt, dass nur die Verwaltungsdaten in dem zweiten Datenträger und nur die aktualisierten Zustandsdaten in dem ersten Datenträger abgespeichert werden.

Die Erfindung ermöglicht eine selbsttätige Abspeicherung der relevanten Daten (Zustandsdaten, Verwaltungsdaten) in dem ersten bzw. zweiten Datenträger.

Vorzugsweise sind die Verwaltungsdaten eines Implantats auf dem ersten Datenträger gespeichert und werden mittels des Steuerprogramms unmittelbar nach der Implantation bzw. anlässlich des erstmaligen Beschreibens des ersten Datenträgers an den zweiten Datenträger übertragen und dort abgespeichert. Dabei werden die Daten in einem Datengerät zwischengespeichert. Diese sich ansammelnden Verwaltungsdaten über die Implantate können dann in bestimmten Abständen von der autorisierten Person zu Abrechnungszwecken aus dem zweiten Datenträger herausgelesen und weiterverarbeitet werden. Vorteilhaft dient das Datengerät zur Steuerung der Kommunikation bzw. zur Vermittlung bei der Kommunikation zwischen dem ersten und zweiten Datenträger sowie zwischen diesen und dem Implantat.

Vorteilhaft dient ein einziges Datengerät zur Verarbeitung der Daten, wobei die Zustandsdaten und die Verwaltungsdaten in unterschiedlichen Speicherbereichen zwischen- oder abgespeichert werden. Durch das Vorhandensein einer Telemetrieeinheit wird eine Kommunikation zwischen dem Datengerät und dem Implantat ermöglicht, wobei die aktualisierten bzw. neuen Daten schnell auf den entsprechenden Datenträgern abgespeichert werden können.

Nach einer Weiterbildung der Erfindung weist das Datengerät ein Kartenlesegerät auf, dem zwei Chipkarten zur jeweiligen Speicherung der Zustandsdaten einerseits und der Verwaltungsdaten andererseits zugeordnet sind. Vorzugsweise weist das Kartenlesegerät eine PCMCIA-Schnittstelle auf, so dass es platzsparend in dem Datengerät integriert ist.

Vorteilhafterweise ist das Datengerät als ein tragbares Datengerät (Laptop) und der Kartenleser als PCMCIA-Kartenleser mit einer Aufnahme für eine Chipkarte in Standardgrösse (Kreditkartengrösse) einerseits und mit einer Aufnahme für eine Chipkarte in Minichipkartenformat (GSM-Karte) ausgebildet. Hierdurch wird das Vorhandensein eines zweiten grossvolumigen Kartenschlitzes eingespart.

Ferner liegt der Erfindung die Aufgabe zugrunde, ein Verfahren zur Verarbeitung von Daten zwischen einem Datengerät und einem Implantat anzugeben, so dass die Datenverarbeitung und - auswertung verbessert wird.

Zur Lösung der Aufgabe weist das erfindungsgemässe Verfahren die Merkmale des Patentanspruchs 9 auf.

Der Vorteil des Verfahrens ist darin zu sehen, dass die Zustandsdaten und die Verwaltungsdaten unabhängig voneinander in dem Datengerät verarbeitet werden und entsprechend den Erfordernissen auf einem jeweiligen Datenträger abgespeichert werden. Hierdurch können die Daten bei Vorliegen tragbarer Datenträger in gesicherter Form aus dem Datengerät entnommen werden und gegebenenfalls einem weiteren Datengerät zugeführt werden zur weiteren Verarbeitung der Daten.

Ein Ausführungsbeispiel der Erfindung wird nachfolgend anhand der Zeichnung näher erläutert.

Es zeigen:
- Figur 1: ein Blockschaltbild eines Datengeräts und
- Figur 2: eine Ausführungsform eines Datengeräts.

Das Datengerät kann zur Verarbeitung von Daten jeglicher Implantate eingesetzt werden. Beispielsweise ist die Verarbeitung von Daten in Herzschrittmachersystemen, Defibrilatoren oder Neurostimulatoren vorgesehen. Vorzugsweise wird dieses Datengerät eingesetzt bei Implantaten, die in regelmässigen Abständen kontrolliert bzw. neu eingestellt werden müssen.

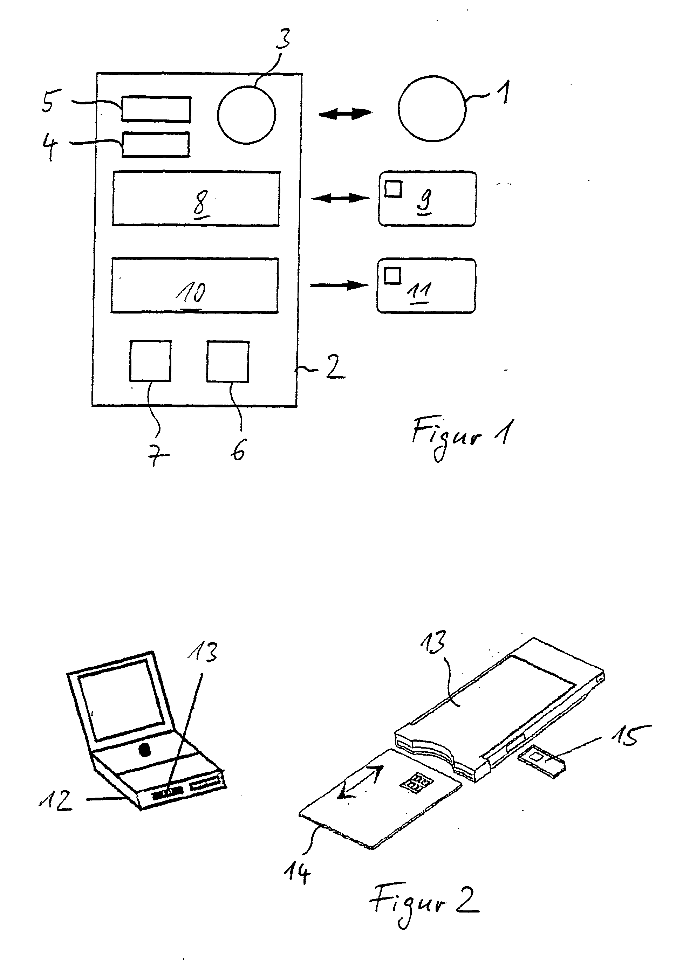

Figur 1 zeigt ein Blockschaltbild eines Datengeräts für einen Herzschrittmacher als Implantat 1, das mit einer nicht dargestellten Steuereinheit zur Steuerung der mittels einer Elektrode in dem Herzbereich abgegebenen elektrischen Impulse ausgestattet ist. Die Impulse weisen eine Reihe von Parametern, wie z.B. Impulsbreite oder Impulshöhe auf, die von der Steuereinheit des Implantats 1 vorgegeben werden. Die Steuerung der Impulse erfolgt in Abhängigkeit von weiteren Einstellparametern, um eine an den medizinischen Befund des Patienten bezogene Anpassung zu gewährleisten.

Die Steuereinheit des Implantats 1 weist zur Kommunikation mit einem externen Datengerät 2 eine nicht dargestellt Spule als Antenne auf, mit der eine Telemetrieeinheit 3 des Datengeräts 2 in Verbindung gebracht werden kann zur Übertragung von Daten in der einen oder der anderen Richtung. Die Telemetrieeinheit 3 weist ebenfalls eine Spule als Antenne auf.

Zur Eingabe der durch telemetrische Übertragung an das Implantat 1 übermittelten Daten weist das Datengerät 2 eine Eingabeeinheit 4 auf, die vorzugsweise als Tastatur ausgebildet ist. Die eingegebenen Daten werden zur Kontrolle in einer Anzeigeeinheit 5 dargestellt. Die Anzeigeeinheit 5 kann als LC-Display oder als Monitor eines Personal Computers oder Laptops ausgebildet sein. Diese Anzeigeeinheit 5 dient auch zur Darstellung der entsprechenden Daten, die aus dem Implantat 1 herausgelesen werden.

Das Datengerät 2 weist eine Steuereinheit 6 und eine Speichereinheit 7 auf, wobei die Steuereinheit 6, durch ein Programm gesteuert, die eingegebenen Daten, insbesondere die zur Einstellung des Implantats 1 erforderlichen Zustandsdaten, verarbeitet und in der Speichereinheit 7 zwischenspeichert. Nachfolgend bewirkt die Steuereinheit 6, dass die Zustandsdaten der Telemetrieeinheit 3 zugeführt werden zur Übertragung an das Implantat 1. Sowohl die Steuereinheit 6 als auch das Implantat 1 weisen einen Mikroprozessor zur Verarbeitung der Daten auf. Vorteilhafterweise ist die Telemetrieeinheit 3 in dem Datengerät 2 integriert, so dass das Datengerät 2 einen kompakten Aufbau aufweist und als tragbares Datengerät 2 vorteilhaft während der Übertragungszeit in die Nähe des Implantats 1 gehalten wird.

Das Implantat 1 weist einen solchen Speicher auf, dass zum einen implantatsbezogene und unveränderbare Daten als sogenannte Verwaltungsdaten und zum anderen patientenbezogene Daten abgespeichert werden können. Die patientenbezogenen Daten bestehen aus unveränderlichen Daten wie Patientencode und Geburtsdatum einerseits und aus durch den Arzt veränderbare Zustandsdaten andererseits. Die veränderbaren Zustandsdaten sind entweder durch den Arzt veränderbare Einstellparameter des Implantats oder Messdaten über den Krankheitszustand des Patienten.

Das Datengerät 2 weist ferner zwei Kartenlesegeräte auf, wobei das erste Kartenlesegerät 8 zu einer ersten Chipkarte (Patientenkarte) 9 und das zweite Kartenlesegerät 10 zu einer zweiten Chipkarte (Abrechnungskarte) 11 korrespondiert. Die Patientenkarte 9 dient zur Abspeicherung von medizinischen Zustandsdaten des Implantats 1 und wird nach der Implantation dem Patienten ausgehändigt. Der Patient führt diese Patientenkarte 9 quasi als Ausweis mit sich und legt diese bei der nächsten Untersuchung dem behandelnden Arzt vor. Durch Einstecken der Patientenkarte 9 in das erste Kartenlesegerät 8 und Ablauf einer softwaregestützten Routine ist es möglich, die aktuellen Zustandsdaten in der Anzeigeeinheit 5 anzuzeigen oder mittels eines angeschlossenen Druckers auszudrucken. Erforderlichenfalls können die Zustandsdaten aufgrund eines medizinischen Befundes geändert werden, wobei die geänderten Zustandsdaten als aktuelle Zustandsdaten zum einen zur Einstellung des Implantats 1 an dasselbe übertragen und zum anderen auf der Patientenkarte 9 abgespeichert werden.

Die Steuereinheit 6 des Datengeräts 2 ermöglicht die selektive Verbindung bzw. Datenübertragung von der Eingabeeinheit 4 bzw. der Telemetrieeinheit 3 zu oder von der Patientenkarte 9 einerseits oder der Abrechnungskarte 10 andererseits. Somit können die aktuellen Zustandsdaten aus der Patientenkarte 9 ausgelesen und der Anzeigeeinheit 5 oder einem angeschlossenen Drucker als Ausgabeeinheit zugeführt werden. Ferner können aktuell mittels der Eingabeeinheit 4 eingegebene Zustandsdaten in der Patientenkarte 9 abgespeichert werden.

Vorzugsweise sind in der Patientenkarte 9 auch herstellerspezifische Daten als Verwaltungsdaten abgespeichert, wie beispielsweise der Herstellername des Implantats, der Modelltyp des Implantats, die Seriennummer, der Bateriezustand des Implantats sowie der Status der Schrittmacherprogrammierung. Die Oberfläche der Patientenkarte 9 weist neben der Kontaktfläche, unterhalb der der Chip in dem Kartenkörper eingebettet ist, noch drucktechnisch aufgebrachte Informationen über das Implantat bzw. des Herstellers auf. Die andere Oberfläche kann als Werbeträger dienen.

Nach einer Ausführungsform der Erfindung können die patientenspezifischen Zustandsdaten auch von dem Implantat 1 selbstätig über ein entsprechendes Steuerprogramm auf die Patientenkarte 9 abgespeichert werden. Hierbei braucht die behandelnde Person lediglich die implantatrelevanten Daten zur Einstellung des Implantats 1 eingeben, wobei nach Überprüfung der korrekten Abspeicherung der Zustandsdaten im Implantat 1 diese der Patientenkarte 9 zugeführt werden. Hierdurch wird sichergestellt, dass allein die dem Implantat zugeführten Daten auf der Patientenkarte 9 abgespeichert werden und keine Fehleingabe von Daten infolge von Verwechslung der Patientendaten eintritt.

Nach einer bevorzugten Ausführungsform der Erfindung werden die auf der Patientenkarte 9 abgespeicherten Verwaltungsdaten genau einmal, und zwar nach erstmaligen Beschreiben der Patientenkarte 9, mittels eines in der Speichereinheit 7 gespeicherten Steuerprogramms auf die Abrechnungskarte 10 geschrieben. Diese Abrechnungskarte 10 ist vorzugsweise ständig mit dem Datengerät 2 verbunden und sammelt die Verwaltungsdaten einer Mehrzahl von mittels des Datengeräts 2 beschriebener Patientenkarten 9. Hierdurch können mittels Ablauf eines einzigen Steuerprogramms die Patientenkarte 9 mit den Zustandsdaten einerseits und die Abrechnungskarte 10 mit den Verwaltungsdaten des entsprechenden Implantats 1 beschrieben werden.

Alternativ können die Verwaltungsdaten auch unmittelbar nach der erstmaligen Eingabe der Zustandsdaten in dem Datengerät 2 in der Abrechnungskarte 10 abgespeichert werden. Hierdurch wird gewährleistet, dass die Abrechnungskarte 10 bereits vor Ausgabe der Patientenkarte 9 beschrieben wird, so dass eine Abrechnung des Implantats 1 sichergestellt ist. Das Steuerprogramm weist Mittel auf, so dass sichergestellt ist, dass die Verwaltungsdaten eines Implantats 1 jeweils nur einmalig in der Abrechnungskarte 10 gespeichert werden.

Die Abrechnungskarte 11 ist dem Datengerät 2 zugeordnet und bleibt im Unterschied zur Patientenkarte 9 im eingesteckten Zustand. Auf der Abrechnungskarte 11 werden bei Vornahme der Implantation die das Implantat 1 kennzeichnenden Daten, wie beispielsweise die Seriennummer, als Verwaltungsdaten abgespeichert. Sie ermöglicht, dass in regelmässigen Abständen die Verwaltungsdaten schnell und sicher durch einen Vertreter des Implantatsherstellers abgerufen werden können, ohne dass der verwaltungstechnische Ablauf der Klinik gestört wird. Vorzugsweise dienen die Verwaltungsdaten zur Abrechnung der Implantate 1 mit der Klinikverwaltung. Die Verwaltungsdaten sind ursprünglich im Implantat 1 gespeichert und werden unter Zwischenspeicherung in dem Datengerät 1 über dasselbe in die Abrechnungskarte 11 eingeschrieben.

Nach einer Ausführungsform der Erfindung gemäss Figur 2 ist ein Datengerät als handelsübliches Notebook 12 ausgebildet, das mit einem PCMCIA-Kartenlesegerät 13 ausgestattet ist. Das PCMCIA-Kartenlesegerät 13 ist geeignet, eine kreditkartengrosse Patientenkarte 14 und eine minichipkartengrosse Abrechnungskarte 15 platzsparend aufzunehmen. Da die Abrechnungskarte 15 nur selten aus dem Datengerät 12 entnommen werden muss, kann sie als Plug-in-Karte ausgebildet sein. Hierdurch wird eine platzsparende Nutzung von zwei Karten ermöglicht.

Vorzugsweise erfolgt das Beschreiben der Patientenkarte 9 bzw. der Abrechnungskarte 11 ausschliesslich in Verbindung mit dem Datengerät 2. Dies wird erzielt durch einen softwaremässig durchgeführten Authentisierungsablauf, in dem die Kennung der Patienten- oder Abrechnungskarte einerseits und des Datengeräts andererseits überprüft wird. Wird durch Vergleich die Berechtigung der Karte festgestellt (beispielsweise durch Eingabe einer PIN), kann ein Datenaustausch erfolgen. Andernfalls kann keine Kommunikation zu oder von den Karten aufgebaut werden.

## Patentansprüche

1. Datengerät (2) mit einem ersten und einem zweiten Datenträger zur telemetrischen Einstellung eines Implantats (1) und/oder zur telemetrischen Kommunikation zwischen dem Implantat (1) und dem Datengerät (2), wobei das Datengerät (2)
- eine Steuereinheit (6) zur Verarbeitung der eingegebenen Daten,
- eine Speichereinheit (7) zur Speicherung der zugeführten Daten,
- eine Eingabeeinheit (4) zur Eingabe von Daten,
- eine Anzeigeeinheit (5) zur Anzeige von Daten,
- eine Telemetrieeinheit (3) zur Übertragung von Daten zum Implantat (1) oder vice versa und **dadurch gekennzeichnet, dass** das Datengerät (2) einen ersten Datenträgerleser (8) zum Lesen und/oder Beschreiben des ersten Datenträgers (9) aufweist, und
einen zweiten Datenträgerleser (10) zum Lesen und/oder Beschreiben des zweiten Datenträgers (11) aufweist,
wobei in dem ersten Datenträger (9) an das Implantat (1) übertragene patentientenbezogene Zustandsdaten und in dem zweiten Datenträger (11) das Implantat (1) betreffende Verwaltungsdaten abgespeichert werden.

2. Datengerät (2) nach Anspruch 1, **dadurch gekennzeichnet, dass** das Datengerät (2) einen ersten Speicherbereich zum Abspeichern der Zustandsdaten und einen zweiten Speicherbereich zum Abspeichern der Verwaltungsdaten aufweist.

3. Datengerät (2) nach Anspruch 1 oder 2, **dadurch gekennzeichnet, dass** die Steuereinheit (6) Mittel aufweist, derart, dass mit dem erstmaligen Abspeichern der Zustandsdaten die Verwaltungsdaten auf dem zweiten Datenträger (11) abspeicherbar sind.

4. Datengerät (2) nach einem der Ansprüche 1 bis 3, **dadurch gekennzeichnet, dass** die Datenträgerleser (8, 10) als Kartenleser (8, 10) und der Datenträger (9, 11) als Chipkarte (9, 11) mit einem Mikroprozessor ausgebildet sind.

5. Datengerät (2) nach Anspruch 4, **dadurch gekennzeichnet, dass** die Kartenleser (8,10) in einem Kartenlesegerät (13) mit einer PCMCIA-Schnittstelle integriert sind, wobei dieses in dem Datengerät (2) integriert ist.

6. Datengerät (2) nach einem der Ansprüche 1 bis 5, **dadurch gekennzeichnet, dass** softwarebasierende Funktionen, die in dem Implantat gespeichert sind, aktivierbar sind unter Bildung eines Aktivierungssignals und dass in Abhängigkeit von dem Aktivierungssignal weitere Informationen als Verwaltungsdaten in dem zweiten Datenträger (11) abspeicherbar sind.

7. Datengerät (2) nach einem der Ansprüche 1 bis 6, **dadurch gekennzeichnet, dass** eine Neufassung oder Erweiterung von einer softwarebasierenden Funktion in das Implantat unter Bildung eines in den zweiten Datenträger abgespeicherten Kennungssignals als Verwaltungsdatum ladbar ist.

8. Datengerät (2) nach einem der Ansprüche 1 bis 7, **dadurch gekennzeichnet, dass** ein Speicher des zweiten Datenträgers (11) derart ausgebildet ist, dass die Verwaltungsdaten mehrerer Implantate speicherbar sind.

9. Verfahren zur Verarbeitung von Daten zwischen einem Datengerät (2) nach Anspruch 1 und einem Implantat (1), **dadurch gekennzeichnet,**
**dass** dem Datengerät (2) der zweite Datenträger (11) zum Abspeichern der implantatsbezogenen Verwaltungsdaten zugeordnet ist, und
**dass** nach Veränderung der Zustandsdaten im Datengerät die aktuellen Zustandsdaten an das Implantat (1) einerseits und an den ersten Datenträger (9) andererseits übertragen und in diesen abgespeichert werden

10. Verfahren nach Anspruch 9, **dadurch gekennzeichnet, dass** die Verwaltungsdaten auf dem zweiten Datenträger (11) abgespeichert werden, sobald die Zustandsdaten erstmalig in dem Datengerät (2) und/oder dem ersten Datenträger (9) abgespeichert werden.

11. Verfahren nach Anspruch 9 oder 10, **dadurch gekennzeichnet, dass** die Verwaltungsdaten nur jeweils an ein einziges Datengerät (2) übertragen und/oder in dem Datengerät (2) abgespeichert werden und dass die Verwaltungsdaten dann einmalig in dem zweiten Datenträger (11) abgespeichert werden.

12. Verfahren nach einem der Ansprüche 9 bis 11, **dadurch gekennzeichnet, dass** die Verwaltungsdaten zumindest bei erstmaligem Gebrauch des ersten Datenträgers (9) von dem ersten Datenträger (9) über das Datengerät (2) zu dem zweiten Datenträger (11) übertragen und dort abgespeichert werden.

13. Verfahren nach einem der Ansprüche 9 bis 11, **dadurch gekennzeichnet, dass** die Verwaltungsdaten in dem Implantat (1) gespeichert werden und telemetrisch an das Datengerät (2) übertragen werden, um dann in einem gesonderten mit dem Datengerät (2) verbindbaren tragbaren Datenträger (11) abgespeichert zu werden.

14. Verfahren nach einem der Ansprüche 9 bis 13, **dadurch gekennzeichnet, dass** die Zustandsdaten zuerst im Implantat (1) und dann in dem ersten Datenträger (9) abgespeichert werden.

## Claims

1. A data device (2) comprising a first and a second data carrier for telemetric setting of an implant (1) and/or for telemetric communication between the implant (1) and the data device (2), wherein the data device (2) has
- a control unit (6) for processing the input data,
- a memory unit (7) for storing the fed data,
- an input unit (4) for inputting data,
- a display unit (5) for displaying data,
- a telemetry unit (3) for transmitting data to the implant (1) or vice versa, and
**characterised in that** the data device (2) has a first data carrier reader (8) for reading from and/or writing to the first data carrier (9) and has a second data carrier reader (10) for reading from and/or writing to the second data carrier (11),
wherein patient-related status data transmitted to the implant (1) is stored in the first data carrier (9) and administrative data concerning the implant (1) is stored in the second data carrier (11).

2. The data device (2) according to Claim 1, **characterised in that** the data device (2) has a first memory region for storing the status data and a second memory region for storing the administrative data.

3. The data device (2) according to Claim 1 or 2, **characterised in that** the control unit (6) has means, such that the administrative data can be stored on the second data carrier (11) when the status data is stored for the first time.

4. The data device (2) according to one of Claims 1 to 3, **characterised in that** the data carrier reader (8, 10) is formed as a card reader (8, 10) and the data carrier (9, 11) is formed as a chip card (9, 11) with a microprocessor.

5. The data device (2) according to Claim 4, **characterised in that** the card readers (8, 10) are integrated in a card reading device (13) with a PCMCIA interface, wherein this is integrated in the data device (2).

6. The data device (2) according to one of Claims 1 to 5, **characterised in that** software-based functions that are stored in the implant can be activated with formation of an activation signal, and **in that** further information can be stored as administrative data in the second data carrier (11) depending on the activation signal.

7. The data device (2) according to one of Claims 1 to 6, **characterised in that** a revision or expansion of a software-based function can be loaded into the implant as administrative data item with formation of an identification signal stored in the second data carrier.

8. The data device (2) according to one of Claims 1 to 7, **characterised in that** a memory of the second data carrier (11) is formed in such a way that the administrative data can be stored in a number of implants.

9. A method for processing data between a data device (2) according to Claim 1 and an implant (1), **characterised in that**
the second data carrier (11) is assigned to the data device (2) in order to store the implant-based administrative data, and
**in that**, once the status data in the data device has been changed, the current status data is transmitted to the implant (1) on the one hand and to the first data carrier (9) on the other hand and is stored therein.

10. The method according to Claim 9, **characterised in that** the administrative data is stored on the second data carrier (11) as soon as the status data has been stored for the first time in the data device (2) and/or the first data carrier (9).

11. The method according to Claim 9 or 10, **characterised in that** the administrative data is transmitted only to a single data device (2) and/or is stored in the data device (2), and **in that** the administrative data is then stored once in the second data carrier (11).

12. The method according to one of Claims 9 to 11, **characterised in that** the administrative data, at least with first-time use of the first data carrier (9), is transmitted from the first data carrier (9) via the data device (2) to the second data carrier (11) and is stored there.

13. The method according to one of Claims 9 to 11, **characterised in that** the administrative data is stored in the implant (1) and is transmitted telemetrically to the data device (2) so as to then be stored in a portable data carrier (11) connectable separately to the data device (2).

14. The method according to one of Claims 9 to 13, **characterised in that** the status data is stored first in the implant (1) and then in the first data carrier (9).

## Revendications

1. Appareil de données (2) avec un premier et un deuxième support de données, pour le réglage télémétrique d'un implant (1) et/ou pour la communication télémétrique entre l'implant (1) et l'appareil de données (2), dans lequel l'appareil de données (2) comporte
- une unité de commande (6) pour le traitement des données entrées,
- une mémoire (7) pour l'enregistrement des données fournies,
- une unité d'entrée (4) pour l'entrée de données,
- une unité d'affichage (5) pour l'affichage de données,
- une unité de télémétrie (3) pour la transmission de données à l'implant (1) ou vice versa, et
**caractérisé en ce que** l'appareil de données (2) comporte un premier lecteur de support de données (8) pour la lecture et/ou la description du premier support de données (9), et un deuxième lecteur de support de données (10) pour la lecture et/ou la description du deuxième support de données (11),
dans lequel des données d'état relatives à un patient transmises à l'implant (1) sont enregistrées sur le premier support de données (9) et des données de gestion concernant l'implant (1) sont enregistrées dans le deuxième support de données (11).

2. Appareil de données (2) selon la revendication 1, **caractérisé en ce que** l'appareil de données (2) comporte une première zone de mémoire pour l'enregistrement des données d'état et une deuxième zone de mémoire pour l'enregistrement des données de gestion.

3. Appareil de données (2) selon la revendication 1 ou 2, **caractérisé en ce que** l'unité de commande (6) comporte des moyens, de sorte que lors du premier enregistrement des données d'état, les données de gestion peuvent être enregistrées sur le deuxième support de données (11).

4. Appareil de données (2) selon l'une des revendications 1 à 3, **caractérisé en ce que** les lecteurs de support de données (8, 10) sont conçus comme des lecteurs de carte (8, 10) et le support de données (9, 11) est conçu comme une carte à puce (9, 11) avec un microprocesseur.

5. Appareil de données (2) selon la revendication 4, **caractérisé en ce que** les lecteurs de carte (8, 10) sont intégrés dans un appareil de lecture de cartes (13) avec une interface PCMCIA, celle-ci étant intégrée dans l'appareil de données (2).

6. Appareil de données (2) selon l'une des revendications 1 à 5, **caractérisé en ce que** des fonctions logicielles enregistrées dans l'implant peuvent être activées par la formation d'un signal d'activation, et **en ce que** d'autres informations peuvent être enregistrées en tant que données de gestion sur le deuxième support de données (11) en fonction du signal d'activation.

7. Appareil de données (2) selon l'une des revendications 1 à 6, **caractérisé en ce qu'**une nouvelle version ou une extension d'une fonction logicielle peut être chargée dans l'implant, par la formation d'un signal d'identification enregistré sur le deuxième support de données, en tant que donnée de gestion.

8. Appareil de données (2) selon l'une des revendications 1 à 7, **caractérisé en ce qu'**une mémoire du deuxième support de données (11) est conçue de manière à pouvoir enregistrer les données de gestion de plusieurs implants.

9. Procédé pour le traitement de données entre un appareil de données (2) selon la revendication 1 et un implant (1), **caractérisé en ce que**
le deuxième support de données (11) est attribué à l'appareil de données (2) pour l'enregistrement de données de gestion relatives à l'implant, et
après la modification des données d'état dans l'appareil de données, les données d'état actuelles sont transmises d'une part à l'implant (1) et d'autre part au premier support de données (9) et enregistrées dans ceux-ci.

10. Procédé selon la revendication 9, **caractérisé en ce que** les données de gestion sont enregistrées sur le deuxième support de données (11) dès que les données d'état sont enregistrées pour la première fois dans l'appareil de données (2) et/ou sur le premier support de données (9).

11. Procédé selon la revendication 9 ou 10, **caractérisé en ce que** les données de gestion sont respectivement transmises à un seul appareil de données (2) et/ou enregistrées dans l'appareil de données (2), et **en ce que** les données de gestion sont alors enregistrées une seule fois sur le deuxième support de données (11).

12. Procédé selon l'une des revendications 9 à 11, **caractérisé en ce que** les données de gestion sont transmises au moins lors de la première utilisation du premier support de données (9), du premier support de données (9) par le biais de l'appareil de données (2) au deuxième support de données (11), et enregistrées sur ce dernier.

13. Procédé selon l'une des revendications 9 à 11, **caractérisé en ce que** les données de gestion sont enregistrées dans l'implant (1) et transmises de façon télémétrique à l'appareil de données (2), pour ensuite être enregistrées sur un support de données portatif (11) apte à être relié individuellement à l'appareil de données (2).

14. Procédé selon l'une des revendications 9 à 13, **caractérisé en ce que** les données d'état sont enregistrées tout d'abord dans l'implant (1) puis sur le premier support de données (9).
